# EUROPEAN PATENT APPLICATION

(11) **EP 4 781 994 A1**
(43) Date of publication of application: **29.07.2026**
(21) Application number: 24894601.4
(22) Date of filing: 20.11.2024
(51) Int. Cl.: A61K 31/65, A61K 31/4164, A61K 9/06, A61K 47/10, A61K 47/14, A61P 1/02

(54) **COMPOSITION FOR PREVENTION OR TREATMENT OF PERIODONTAL DISEASE AND DENTAL COMPOSITE ANTIBIOTIC OINTMENT COMPOSITION**

(30) Priority: 22.11.2023 KR 20230163752; 19.11.2024 KR 20240165615
(71) Applicant: MX BIO INC., Seoul 08589 (KR)
(72) Inventor: LEE, Jae Hyun, Seongnam-si Gyeonggi-do 13554 (KR); HONG, SeongIk, Seoul 07048 (KR); SHIN, Minji, Seoul 08395 (KR)
(74) Representative: RGTH
(86) International application number: PCT/KR2024/018379
(87) International publication number: WO 2025/110709

(57) **Abstract**

The present invention relates to a composition for the prevention or treatment of periodontal disease and a dental composite antibiotic ointment composition. The composition for the prevention or treatment of periodontal disease according to the present invention comprises minocycline and metronidazole as active ingredients.

## Description

### [Technical Field]

The present invention relates to a composition for the prevention or treatment of periodontal disease and a dental composite antibiotic ointment composition.

### [Background Art]

Periodontal disease is caused by bacterial infection due to the failure of maintaining oral hygiene in the gums, teeth, and implant sites. Periodontal disease includes periodontitis, which occurs mainly in natural teeth, and peri-implantitis, which occurs in sites where dental implants have been implanted. Periodontitis is accompanied by symptoms such as increased depth and bleeding of the paradental cyst, gum erosion, bone loss, etc., when progressing to severe disease.

Peri-implantitis is similar to periodontitis. However, compared to periodontitis, the lesion extends to the periapical site and shows symptoms of acute inflammation. In addition, the oral microbial distribution of periodontitis is different from the microbial distribution of periodontitis, and redness, edema, mucosal hypertrophy, abscess, bleeding, an increased depth of the paradental cyst, and an increase in bone loss occur in the implant sites.

There is a difference in the symptoms of periodontitis and peri-implantitis, but the treatment method thereof may be divided into surgical treatment and non-surgical treatment, and treatment may be performed similarly for each disease. Surgically, periodontitis may undergo treatment for removing inflammation and tartar plaque and remodeling the gingival contour through a procedure such as scaling and denture removal and root planning after gum incision. Peri-implantitis may be treated by removing inflammation similarly to periodontitis, and may be undergone a process of removing biofilm on the surface of the implant using a laser, a nickel-titanium brush or the like. Both periodontal diseases may go through a process of carrying out bone transplantation and thus repairing the lost gum bone, if it is necessary.

Non-surgically, both periodontitis and peri-implantitis undergo treatment using antibiotics, and also undergo non-surgical treatment by using existing dental antibiotic ointments containing minocycline hydrochloride serving as a main component. Dental ointments are directly injected between the paradental cysts after removing tartar, dental plaque, inflammation, etc., and are performed as a local treatment. There are many clinical cases in which dental ointments currently on the market have an effect on periodontitis.

However, in the treatment of peri-implantitis, existing single-component dental ointments are difficult to treat all oral microorganisms for the corresponding symptoms and have pathological differences, and thus the existing treatment drugs have limitations to their application as a clear non-surgical treatment method for peri-implantitis, thereby urging a need for applying other drugs.

Therefore, according to the present invention, an antibiotic ointment composition using a combination of two antibiotics has been developed in order to solve the problem derived from dental ointments using conventional single-component antibiotics. Accordingly, the present invention is intended to enable the use of a non-surgical therapeutic agent which is capable of more effectively treating periodontal disease such as periodontitis and peri-implantitis than the existing single-component dental ointments.

Patent Document 1: Persson GR, Renvert S. Cluster of bacteria associated with peri-implantitis. Clin Implant Dent Relat Res. 2014 Dec;16(6):783-93. doi: 10.1111/cid.12052. Epub 2013 Mar 25. PMID: 23527870*.*

Patent Document 2: Maruyama, N., Maruyama, F., Takeuchi, Y. et al. Intraindividual variation in core microbiota in peri-implantitis and periodontitis. Sci Rep 4, 6602 (2014*).*

Patent Document 3: Heitz-Mayfield LJ, Lang NP. Surgical and nonsurgical periodontal therapy. Learned and unlearned concepts. Periodontal 2000. 2013 Jun;62(1):218-31. doi: 10.1111/prd.12008. PMID: 23574468*.*

Patent Document 4: Korean Registered Patent Publication NO. 10-0252699

Patent Document 5: Berglundh T, Zitzmann NU, Donati M. Are peri-implantitis lesions different from periodontitis lesions?J Clin Periodontol. 2011 Mar;38 Suppl 11:188-202. doi: 10.1111/j.1600-051X.2010.01672.x. PMID: 21323715*.*

Patent Document 6: Becker ST, Beck-Broichsitter BE, Graetz C, Dφrfer CE, Wiltfang J, Hasler R. Peri-implantitis versus periodontitis: functional differences indicated by transcriptome profiling. Clin Implant Dent Relat Res. 2014 Jun;16(3):401-11. doi: 10.1111/cid.12001. Epub 2012 Sep 11. PMID: 22967131*.*

### [Disclosure]

### [Technical Problem]

In order to solve the above problems of the related art, the present inventors have intended to provide a composition for the prevention or treatment of periodontal disease and a dental composite antibiotic ointment composition, including minocycline and metronidazole as active ingredients.

### [Technical Solution]

In order to solve the above-described problems, the present invention may provide a composition for the prevention or treatment of periodontal disease including minocycline and metronidazole as active ingredients.

In addition, the present invention may provide a composition for the prevention or treatment of periodontal disease in which the minocycline includes minocycline hydrochloride and the metronidazole includes metronidazole benzoate as active ingredients.

Furthermore, the present invention may provide a composition for the prevention or treatment of periodontal disease, in which the minocycline and the metronidazole are mixed and used at a weight ratio of 1:1 to 1:25.

Moreover, the present invention may provide a composition for the prevention or treatment of periodontal disease, in which the metronidazole has a particle size (D90) of 5 to 150 µm.

Besides, the present invention may provide a composition for the prevention or treatment of periodontal disease, in which the metronidazole has a bulk density of 0.2 to 0.5 g/cm³.

Further, other embodiments of the present invention may provide a dental composite antibiotic ointment composition including minocycline, metronidazole, a base, an emulsifier, a viscosity modifier, a stabilizer, and an antioxidant.

In addition, the present invention may provide a dental composite antibiotic ointment composition in which the minocycline is used at a ratio of 0.1 to 2 wt% of the total weight of the ointment, and the metronidazole is used at a ratio of 0.1 to 50 wt% of the total weight of the ointment.

Furthermore, the present invention may provide a dental composite antibiotic ointment composition in which the base is used at a ratio of 5 to 25 wt% of the total weight of the ointment and includes at least one selected from the group consisting of concentrated glycerin, polyethylene glycol 400, polyethylene glycol 4000, propylene glycol, 1,3-propanediol, isoprene glycol, pentylene glycol, hexylene glycol, butylene glycol, diethylene glycol, dipropylene glycol, diglycerol, erythritol, arabitol, adonitol, sorbitol, mannitol, xylitol, dulcitol, glucose, fructose, xylose, trehalose, maltose, saccharose, and lactose.

In addition, the present invention may provide a dental composite antibiotic ointment composition in which the emulsifier is used at a ratio of 20 to 50 wt% with respect to the total weight of the ointment and includes at least one selected from the group consisting of glyceryl monostearate, glyceryl monooleate, glyceryl monolaurate, glyceryl monopalmitate, acetylated monoglyceride, white vaseline, liquid paraffin, paraffin, stearic acid, cetostearyl alcohol, isopropylmyristate, and cetylpalmitate.

Besides, the present invention may provide a dental composite antibiotic ointment composition in which the viscosity modifier is used at a ratio of 1 to 20 wt% of the total weight of the ointment and includes at least one selected from the group consisting of mineral oil, sesame oil, tea tree oil, avocado oil, castor oil, canola oil, coffee oil, corn oil, jojoba oil, kaya oil, linseed oil, macadamia oil, olive oil, peanut oil, rapeseed oil, cottonseed oil, rice bran oil, safflower seed oil, camellia oil, sunflower seed oil, soybean oil, shea butter, green tea seed oil, borage oil, walnut oil, evening primrose oil, lanolin oil, calendula oil, rosehip oil, coconut oil, mustard oil, palm olein oil, palm kernel oil, apricot seed oil, grape seed oil, castor oil, phosphide oil, argan oil, linseed oil, almond oil, hazelnut oil, hemp seed oil, and pumpkin seed oil.

Further, the present invention may provide a dental composite antibiotic ointment composition in which the stabilizer is used at a ratio of 1 to 8 wt% of the total weight of the ointment and includes magnesium chloride.

In addition, the present invention may provide a dental composite antibiotic ointment composition in which the antioxidant is used at a ratio of 2 wt% or less of the total weight of the ointment and includes ascorbyl palmitate.

### [Advantageous Effects]

A composition for the prevention or treatment of periodontal disease according to the present invention can have an effect of treating periodontal disease including peri-implantitis or periodontitis, which has not been solved by single-component antibiotics, by applying composite antibiotics thereto and removing bacteria therefrom.

### [Description of Drawings]

The following drawings attached to the present specification illustrate preferred embodiments of the present invention and serve to further understand the technical idea of the present invention together with the detailed description of the present invention, and thus the present invention should not be construed as being limited to the matters described in the drawings.
FIG. 1 is a graph showing the result of testing on a syringe injection force of a dental composite antibiotic ointment composition according to one embodiment of the present invention.
FIGS. 2 and 3 are graphs showing the results of testing on a syringe injection force according to comparative examples of the present invention.
FIG. 4 is a view showing the result of testing on the adhesive properties of a dental composite antibiotic ointment composition according to one embodiment of the present invention and Comparative Example 1, which is a single-component ointment, in artificial saliva.
FIG. 5 is a view showing the viscosity and properties of an ointment composition according to the bulk density of metronidazole benzoate according to one embodiment of the present invention.
FIG. 6 is a graph showing the result of measuring the depth recovery rate of the paradental cyst according to treatment of an ointment composition according to one embodiment of the present invention and Comparative Example 1, which is a single-component ointment.
FIG. 7 is a view showing the measurement of a change in the presence or absence of inflammation according to treatment with an ointment composition according to one embodiment of the present invention and Comparative Example 1, which is a single-component ointment.

### [Best Mode]

In order to solve the above-described problems, the present invention may provide a composition for the prevention or treatment of periodontal disease including minocycline and metronidazole as active ingredients.

### [Mode for Invention]

Hereinafter, embodiments of the present invention will be described in detail with reference to the accompanying drawings. The following embodiments are presented to fully convey the spirit of the present invention to those skilled in the art to which the present invention pertains. The present invention is not limited to the embodiments presented herein and may be embodied in other forms. In the drawings, parts irrelevant to the description may be omitted to clarify the present invention, and sizes of components may be exaggerated to help understanding.

The terminology used in the present specification is only for the purpose of referring to particular embodiments and is not intended to limit the scope of the invention. In addition, the singular forms used herein also include plural forms unless the phrases clearly mean the opposite. Furthermore, the meaning of "comprising" or "containing" used in the present specification specifies a specific property, region, integer, step, operation, element, or component, and does not exclude the addition of another specific property, region, integer, step, operation, element, or component.

In the present invention, terms such as first, second, etc., are used to describe various components, and the terms are used only for the purpose of distinguishing one component from another component.

In addition, terms used in the present specification are used only to describe certain exemplary embodiments and are not intended to limit the present invention. The terms of a singular form may include plural forms unless otherwise specified. In the present specification, the terms "comprise," "include," "have" or the like are intended to designate that the performed features, numbers, steps, components, or combinations thereof are present, and are not construed as excluding the possibility that one or more other features, numbers, steps, components, or combinations thereof may be present or added.

Since the present invention may make various changes and have various forms, specific embodiments will be illustrated and described in detail below. However, they are not intended to limit the present invention to the specific disclosed forms, and it should be understood that the present invention includes all modifications, equivalents, and substitutes included in the spirit and scope of the present invention.

Hereinafter, a composition for the prevention or treatment of periodontal disease and a dental composite antibiotic ointment composition according to the present invention will be sequentially described in detail.

According to one embodiment of the present invention, the composition for the prevention or treatment of periodontal disease may include minocycline and metronidazole as active ingredients.

The minocycline and the metronidazole may be antibiotics and used as components for the prevention or treatment of periodontal disease. Metronidazole as well as minocycline, which has been widely used in the past, may be used in combination to treat bacteria that have not been treated by single-component antibiotics.

For example, the minocycline may include minocycline hydrochloride and the metronidazole may include metronidazole benzoate as active ingredients.
The minocycline and the metronidazole may be mixed and used at a weight ratio of 1:1 to 1:25.

When the mixing ratio of the minocycline and the metronidazole is less than 1:1, the drug concentration may be too low to have a therapeutic effect, and when the concentration exceeds 1:25, there may be a problem in terms of maintaining the properties of the ointment formulation.

The metronidazole may have a particle size (D90) of 5 to 150 µm.

When a particle size of the metronidazole is less than 5 µm, the composition may have too high viscosity, so the composition may agglomerate and fail to achieve a good dispersion therein, so that it is difficult to achieve the effect of the metronidazole, and when the particle size thereof exceeds 150 µm, the composition may have a lower viscosity, thus causing a problem in which a clogging phenomenon occurs when a syringe nozzle (nozzle diameter of 0.4 to 0.6 mm) having a specification applicable to the paradental cyst is applied.

Preferably, the metronidazole may have a particle size (D90) of 5 to 100 µm, the metronidazole may have a particle size (D90) of 10 to 90 µm, and the metronidazole may have a particle size (D90) of 20 to 50 µm.

The metronidazole may have a density of 0.2 to 0.5 g/cm³.

When the metronidazole has a density of less than 0.2 g/cm³, the composition or the ointment may have too high viscosity, thus failing to achieve good spreadability when it is used as an ointment, and when the metronidazole has a density of more than 0.5 g/cm³, the composition or the ointment may have too low a viscosity, thus causing a problem of being not attached to, but detached from the skin without good spreadability when used as an ointment.

The composition for the prevention or treatment of periodontal disease of the present invention may be formulated in the form of an ointment.

The dental composite antibiotic ointment composition according to one embodiment of the present invention may include minocycline, metronidazole, a base, an emulsifier, a viscosity modifier, a stabilizer, and an antioxidant.

The minocycline may be used at a ratio of 0.1 to 2 wt% of the total weight of the ointment, and the metronidazole may be used at a ratio of 0.1 to 50 wt% of the total weight of the ointment, and it may be preferable that the minocycline is added in an effective amount in order to exhibit an effective effect for preventing or treating periodontal disease.

The composition for the prevention or treatment of periodontal disease of the present invention may use a base to be formulated in the form of an ointment.

The base may be used at a ratio of 5 to 25 wt% of the total weight of the ointment and may include at least one selected from the group consisting of concentrated glycerin, polyethylene glycol 400, polyethylene glycol 4000, propylene glycol, 1,3-propanediol, isoprene glycol, pentylene glycol, hexylene glycol, butylene glycol, diethylene glycol, dipropylene glycol, diglycerol, erythritol, arabitol, adonitol, sorbitol, mannitol, xylitol, dulcitol, glucose, fructose, xylose, trehalose, maltose, saccharose, and lactose.

The emulsifier may be used at a ratio of 20 to 50 wt% of the total weight of the ointment and may include at least one selected from the group consisting of glyceryl monostearate, glyceryl monooleate, glyceryl monolaurate, glyceryl monopalmitate, acetylated monoglyceride, white vaseline, liquid paraffin, paraffin, stearic acid, cetostearyl alcohol, isopropylmyristate, and cetylpalmitate.

For example, the base and the emulsifier may be mixed and used at a weight ratio of 1:1 to 1:4.

When the weight ratio of the base and the emulsifier is less than 1:1, the dental composite antibiotic ointment composition may have too high viscosity, thus causing a problem of not being discharged from a syringe, and when the weight ratio of the base and the emulsifier is more than 1:4, the dental composite antibiotic ointment composition may have too low a viscosity, thus causing a problem of being not attached to, but detached from the skin without good spreadability when used as an ointment.

The viscosity modifier may be used at a ratio of 1 to 20 wt% of the total weight of the ointment and may include at least one selected from the group consisting of mineral oil, sesame oil, tea tree oil, avocado oil, castor oil, canola oil, coffee oil, corn oil, jojoba oil, kaya oil, linseed oil, macadamia oil, olive oil, peanut oil, rapeseed oil, cottonseed oil, rice bran oil, safflower seed oil, camellia oil, sunflower seed oil, soybean oil, shea butter, green tea seed oil, borage oil, walnut oil, evening primrose oil, lanolin oil, calendula oil, rosehip oil, coconut oil, mustard oil, palm olein oil, palm oil, palm kernel oil, apricot seed oil, grape seed oil, castor oil, poppyseed oil, argan oil, linseed oil, almond oil, hazelnut oil, hemp seed oil, and pumpkin seed oil.

The stabilizer may be used at a ratio of 1 to 8 wt% of the total weight of the ointment and may include magnesium chloride.

The antioxidant may be used at a ratio of 2 wt% or less of the total weight of the ointment and includes ascorbyl palmitate.

The composition may be preferably a pharmaceutical composition, and more preferably a composition having a formulation in the form of an ointment.

The composition may be provided as a pharmaceutical composition including an active ingredient alone or in combination with one or more pharmaceutically acceptable carriers, excipients, or diluents.

The term "pharmaceutically acceptable" may refer to exhibiting non-toxic properties to cells or humans exposed to the composition.

In addition, the composition may be provided by being mixed with a conventionally known material for treating periodontal disease. In other words, the composition may be administered in combination with a known compound having an effect of preventing or treating periodontal disease.

The term "administration" may refer to introducing a predetermined substance to an individual by an appropriate method, and the term "individual" may mean all animals such as rats, mice, livestock, etc., including humans, who have suffered from or may develop periodontal disease. As a specific example, it may be a mammal including a human.

A preferred dosage of the composition may vary depending on the condition and body weight of a patient, the severity of disease, a drug form, and administration route and period, but may be appropriately selected by those skilled in the art. However, for a preferred effect, the composition may be preferably administered at 0.01 to 1000 mg/kg/day, preferably 0.1 to 500 mg/kg/day, but is not limited thereto. The administration may be performed once a day or divided into several times. The dosage may not be limited in the scope at all.

Hereinafter, the present invention will be described in more detail with reference to examples and experimental examples.

However, the following examples and experimental examples are provided only for the purpose of illustrating the present invention, and thus the content of the present invention is not limited thereto.

### Example 1

A dental composite antibiotic ointment composition was prepared according to the composition ratio as shown in Table 1 below.

**[Table 1]**

| Raw material name | Content (wt%) | Function |
|---|---|---|
| Minocycline hydrochloride | 2.0 | Antibiotics |
| Metronidazole benzoate | 40.2 | Antibiotics |
| Concentrated glycerin | 25.0 | Base |
| Glyceryl monooleate | 25.0 | Emulsifier |
| Sesame oil | 3.0 | Viscosity modifier |
| Magnesium chloride | 4.0 | Stabilizer |
| Ascorbyl palmitate | 0.8 | Antioxidant |
| Total | 100 | - |

As the dental composite antibiotic ointment composition prepared according to above Example 1, each ointment was added into the syringe thereof, so as to measure an injection force thereof. As a comparative example, a syringe injection force of a product of Company A (Comparative Example 1) and a product of Company B (Comparative Example 2), which are commercially available as a single-component product of minocycline, was compared and measured.

In this case, the composition of Comparative Example 1 includes minocycline hydrochloride as a main component, sodium alginate, plastibase, hypromellose 2910 and polysorbate 80 as a base, chitosan as an antioxidant, sodium carboxymethylcellulose and carbomer as a thickener, and the composition of Comparative Example 2 includes minocycline hydrochloride as a main component, hydroxyethylcellulose and ammonio methacrylate copolymers as a sustained-release agent, triacetin as a solubilizer, magnesium chloride as a stabilizer, and concentrated glycerin as a solvent.

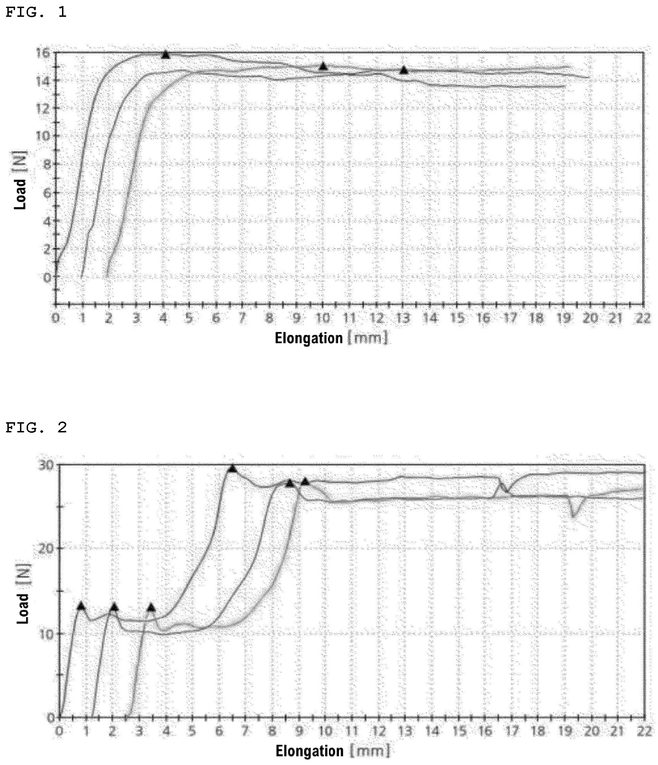

Each plastic syringe container was filled with 1 ml of the dental composite antibiotic ointment composition prepared according to above Example 1 and Comparative Examples 1 and 2. The syringe was mounted on a test holder of a small tabletop precision universal testing machine and a vertical downward force was applied to a syringe pusher using a force gauge at a rate of 100±5 mm/min. The force required to discharge the ointment from the syringe was measured and the tester was stopped at 10% or less of the nominal capacity in the syringe to record the maximum load and confirm whether the ointment is easily discharged from the syringe container. It was determined that the load value of 20 N or less was passed (o), the load value between 20 and 25 N was appropriate (Δ), and the load value exceeding 25 N was rejected (×). Other results are presented in Table 2 and FIGS. 1 to 3 below.

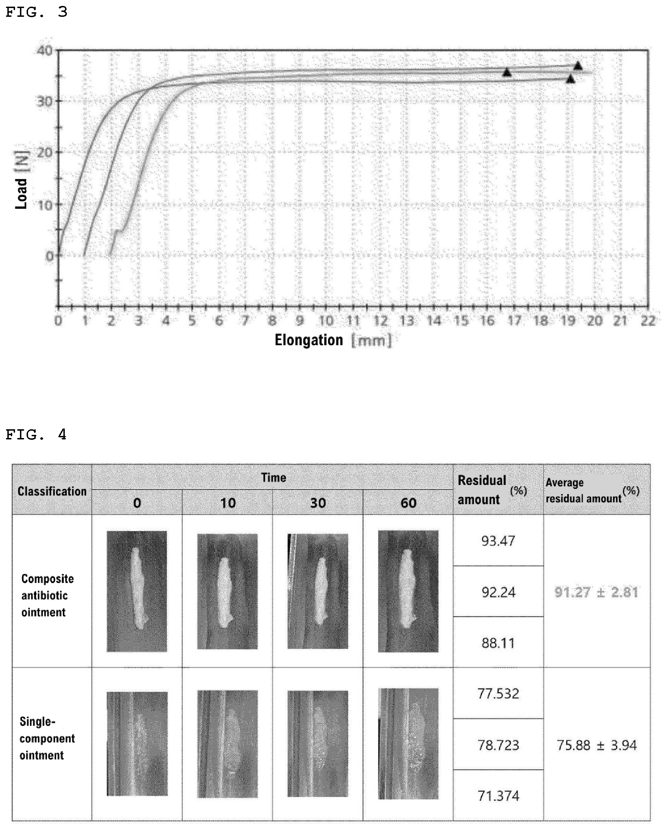

FIG. 1 is a graph showing the result of testing on a syringe injection force of a dental composite antibiotic ointment composition according to one embodiment of the present invention. FIGS. 2 and 3 are graphs showing the results of testing on a syringe injection force according to Comparative Examples 1 and 2 of the present invention.

**[Table 2]**

| | Syringe injection force (N) | Evaluation results |
|---|---|---|
| Example 1 | 15.22 ± 0.57 | O |
| Comparative Example 1 | 28.49 ±. 0.98 | X |
| Comparative Example 2 | 35.74 ± 1.28 | X |

The adhesive properties of the dental composite antibiotic ointment composition prepared according to above Example 1 and Comparative Example 2 as a comparative example in artificial saliva were tested three times, and the results are shown in Table 3 and FIG. 4 below. FIG. 4 is a view showing the result of testing on the adhesive properties of a dental composite antibiotic ointment composition according to one embodiment of the present invention and Comparative Example 1, which is a single-component ointment, in artificial saliva.

**[Table 3]**

| | Residual amount (%) | Average residual amount (%) |
|---|---|---|
| Example 1-1 | 93.47 | 91.27 |
| Example 1-2 | 92.24 | |
| Example 1-3 | 88.11 | |
| Comparative Example 2-1 | 77.532 | 75.88 |
| Comparative Example 2-2 | 78.723 | |
| Comparative Example 2-3 | 71.374 | |

### Examples 2 to 4 and Comparative Examples 1 to 4

A dental composite antibiotic ointment composition was prepared by adjusting the mixing ratio of concentrated glycerin and glyceryl monooleate corresponding to the base and the emulsifier in above Example 1 as shown in Table 4 below.

**[Table 4]**

| | Concentrated glycerin: Weight ratio of glyceryl monooleate |
|---|---|
| Example 1 | 1:1 |
| Example 2 | 1:1.5 |
| Example 3 | 1:4 |
| Comparative Example 3 | Glyceryl monooleate 100% |
| Comparative Example 4 | Concentrated glycerin 100% |
| Comparative Example 5 | 1.5:1 |
| Comparative Example 6 | 4:1 |

In addition, the viscosities of the dental composite antibiotic ointment compositions prepared according to above Examples 1 to 3 and Comparative Examples 3 to 6 were measured, and each ointment was added to the syringe to measure the injection force, which is shown in Table 5 below.

**[Table 5]**

| | Viscosity (cPs) | Result of evaluating syringe injection force |
|---|---|---|
| Example 1 | 10,338.3 ± 289.3 | Δ |
| Example 2 | 7,716.7 ± 278.3 | O |
| Example 3 | 7,391.7 ± 953.3 | O |
| Comparative Example 3 | - | - |
| Comparative Example 4 | 13,877.3 ± 518.7 | X |
| Comparative Example 5 | 13,514.0 ± 1,518 | X |
| Comparative Example 6 | 13,939.3 ± 1,729.3 | X |

### Examples 4 to 6 and Comparative Examples 7 and 8

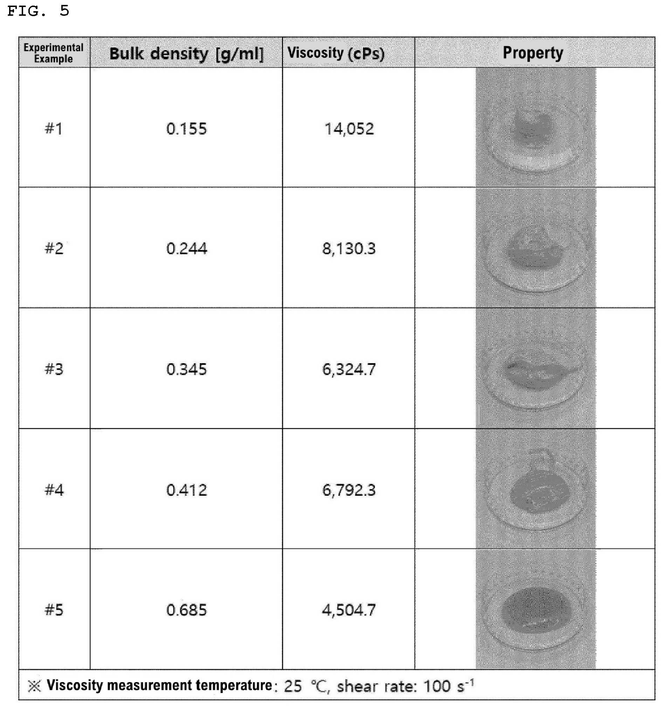

A dental composite antibiotic ointment composition was prepared by adjusting the bulk density of metronidazole benzoate in above Example 1 as shown in Table 6 below, and the viscosity was measured accordingly, and the results are shown in Table 6 and FIG. 5.

FIG. 5 is a view showing the viscosity and properties of an ointment composition according to the bulk density of metronidazole benzoate according to one embodiment of the present invention.

**[Table 6]**

| | Bulk density (g/cm³) | Viscosity (cPs) |
|---|---|---|
| Comparative Example 7 | 0.155 | 14,052 |
| Example 4 | 0.244 | 8,130.3 |
| Example 5 | 0.345 | 6,324.7 |
| Example 6 | 0.412 | 6,792.3 |
| Comparative Example 8 | 0.685 | 4,504.7 |

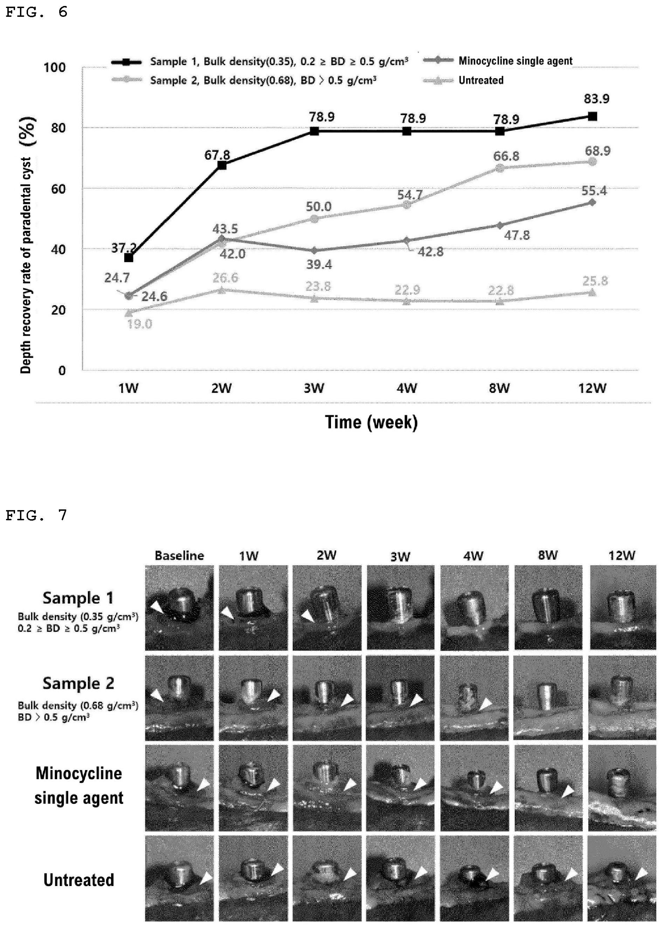

In addition, the depth recovery rate of the paradental cyst according to treatment with the dental composite antibiotic ointment composition prepared according to above Example 5 and Comparative Example 8 was tested, and the results are shown in FIG. 6.

FIG. 6 is a graph showing the result of measuring the depth recovery rate of the paradental cyst according to treatment with an ointment composition according to one embodiment of the present invention and Comparative Example 1, which is a single-component ointment.

Referring to Table 6 and FIG. 6, the bulk density of 0.35 corresponding to Example 5 is shown in a blue graph, and it was found that the depth recovery rate of the paradental cyst rapidly increased over time, resulting that the recovery rate was about 78.90% in three weeks later, and the recovery rate was 83.9% in 12 weeks later.

In contrast, the bulk density of 0.68 corresponding to Comparative Example 8 is shown in a gray graph, and it was found that the recovery rate was 68.9% over time in 12 weeks later, but the depth recovery rate of the paradental cyst was significantly lower than that of Example 5.

In addition, the case of Comparative Example 1, which uses a minocycline single agent, is shown in an orange-colored graph, and it was found that a depth recovery rate of the paradental cyst was more excellent as compared with the case that is not treated with the ointment (which is shown in yellow color), and a recovery rate was lower compared to the composite antibiotic ointment. In particular, a recovery rate was significantly lower compared to Example 5.

In addition, changes in inflammation according to treatment with the dental composite antibiotic ointment compositions prepared according to above Example 5 and Comparative Example 8 were observed, and the results thereof are shown in FIG. 7.

FIG. 7 is a view showing the measurement of a change in the presence or absence of inflammation according to treatment with an ointment composition according to one embodiment of the present invention and Comparative Example 1, which is a single-component ointment.

Referring to Table 6 and FIG. 7, in the case of the bulk density of 0.35 corresponding to Example 5, it could be confirmed that most of the inflammation disappeared in about Weeks 2 to 3, in the case of the bulk density of 0.68 corresponding to Comparative Example 6, it could be confirmed that the inflammation disappeared after Week 4, in the case of Comparative Example 1 used as a minocycline single agent, it could be confirmed that the inflammation disappeared after Week 8, and in the case of the untreated group, it could be confirmed that the inflammation was continuously maintained.

The above description of the present application is for illustrative purposes, and those of ordinary skill in the art to which the present application pertains will understand that the present application may be easily modified into other specific forms without changing the technical spirit or essential features of the present application. Thus, it needs to be understood that the embodiments described above are exemplary and not restrictive in all aspects. For example, each component described as a single type may be distributed and implemented, and similarly, the components described as being distributed may also be implemented in a combined form.

The scope of the present application is presented by the patent claims to be described later rather than the above detailed description and needs to be construed as including all changes or modifications derived from the meaning and scope of the patent claims and the equivalent concept thereof.

## Claims

1. A composition for prevention or treatment of periodontal disease comprising minocycline and metronidazole as active ingredients.

2. The composition of claim 1, wherein the minocycline includes minocycline hydrochloride and the metronidazole includes metronidazole benzoate as active ingredients.

3. The composition of claim 1, wherein the minocycline and the metronidazole are mixed and used at a weight ratio of 1:1 to 1:25.

4. The composition of claim 1, wherein the metronidazole has a particle size (D90) of 5 to 150 µm.

5. The composition of claim 1, wherein the metronidazole has a bulk density of 0.2 to 0.5 g/cm³.

6. A dental composite antibiotic ointment composition comprising minocycline, metronidazole, a base, an emulsifier, a viscosity modifier, a stabilizer, and an antioxidant.

7. The composition of claim 6, wherein the minocycline is used at a ratio of 0.1 to 2 wt% of the total weight of the ointment, and the metronidazole is used at a ratio of 0.1 to 50 wt% of the total weight of the ointment.

8. The composition of claim 6, wherein the base is used at a ratio of 5 to 25 wt% of the total weight of the ointment and includes at least one selected from the group consisting of concentrated glycerin, polyethylene glycol 400, polyethylene glycol 4000, propylene glycol, 1,3-propanediol, isoprene glycol, pentylene glycol, hexylene glycol, butylene glycol, diethylene glycol, dipropylene glycol, diglycerol, erythritol, arabitol, adonitol, sorbitol, mannitol, xylitol, dulcitol, glucose, fructose, xylose, trehalose, maltose, saccharose, and lactose.

9. The composition of claim 6, wherein the emulsifier is used at a ratio of 20 to 50 wt% of the total weight of the ointment and includes at least one selected from the group consisting of glyceryl monostearate, glyceryl monooleate, glyceryl monolaurate, glyceryl monopalmitate, acetylated monoglyceride, white vaseline, liquid paraffin, paraffin, stearic acid, cetostearyl alcohol, isopropylmyristate, and cetylpalmitate.

10. The composition of claim 1, wherein the viscosity modifier is used at a ratio of 1 to 20 wt% of the total weight of the ointment and includes at least one selected from the group consisting of mineral oil, sesame oil, tea tree oil, avocado oil, castor oil, canola oil, coffee oil, corn oil, jojoba oil, kaya oil, linseed oil, macadamia oil, olive oil, peanut oil, rapeseed oil, cottonseed oil, rice bran oil, safflower seed oil, camellia oil, sunflower seed oil, soybean oil, shea butter, green tea seed oil, borage oil, walnut oil, evening primrose oil, lanolin oil, calendula oil, rosehip oil, coconut oil, mustard oil, palm olein oil, palm oil, palm kernel oil, apricot seed oil, grape seed oil, castor oil, poppyseed oil, argan oil, linseed oil, almond oil, hazelnut oil, hemp seed oil, and pumpkin seed oil.

11. The composition of claim 1, wherein the stabilizer is used at a ratio of 1 to 8 wt% of the total weight of the ointment and includes magnesium chloride.

12. The composition of claim 1, wherein the antioxidant is used at a ratio of 2 wt% or less of the total weight of the ointment and includes ascorbyl palmitate.
